(19) Europäisches Patentamt
European Patent Office
Office européen des brevets

(11) Publication number: **0 311 228 A2**

(12) # EUROPEAN PATENT APPLICATION

(21) Application number: 88302695.7

(22) Date of filing: 25.03.88

(51) Int. Cl.[4]: C07K 13/00 , C12N 15/00 , G01N 33/569 , A61K 37/02 , A61K 39/21 , C12P 21/02

(30) Priority: 09.10.87 US 107703

(43) Date of publication of application: 12.04.89 Bulletin 89/15

(84) Designated Contracting States: AT BE CH DE ES FR GB GR IT LI LU NL SE

(71) Applicant: REPLIGEN CORPORATION
101 Binney Street
Cambridge Massachusetts 02142(US)

(72) Inventor: Putney, Scott D.
5 Epping Street
Arlington MA 02174(US)
Inventor: Lynn, Debra
11 Allen Street Apt. 11
Arlington MA 02174(US)
Inventor: Javaherian, Kashayar
27 Webster Road
Lexington, MA 02173(US)
Inventor: Mueller, William T.
26 Copeland Street
Watertown, MA 02172(US)
Inventor: Farley, John
261 Culver Road, Nr. 9
Rochester, NY 14607(US)

(74) Representative: Perry, Robert Edward et al
GILL JENNINGS & EVERY 53-64 Chancery Lane
London WC2A 1HN(GB)

(54) **Recombinant polypeptides and their uses, including assay for AIDS virus.**

(57) The HIV portions of 4 proteins are useful for the diagnosis of, and in preparing vaccines for, the HIV virus.

EP 0 311 228 A2

# RECOMBINANT POLYPEPTIDES AND THEIR USES, INCLUDING ASSAY FOR AIDS VIRUS

This invention relates to recombinant polypeptides and their uses, including assay for AIDS virus. A full background, and polypeptides which can be used for the same purpose, are given in EP-A-0255190, published 03.02.88.

In particular, EP-A-0255190 discloses proteins indentified as R10, PB1, 590 and KH1. Their amino-acid sequences are given, as are the nucleotide sequences encoding these fusion proteins.

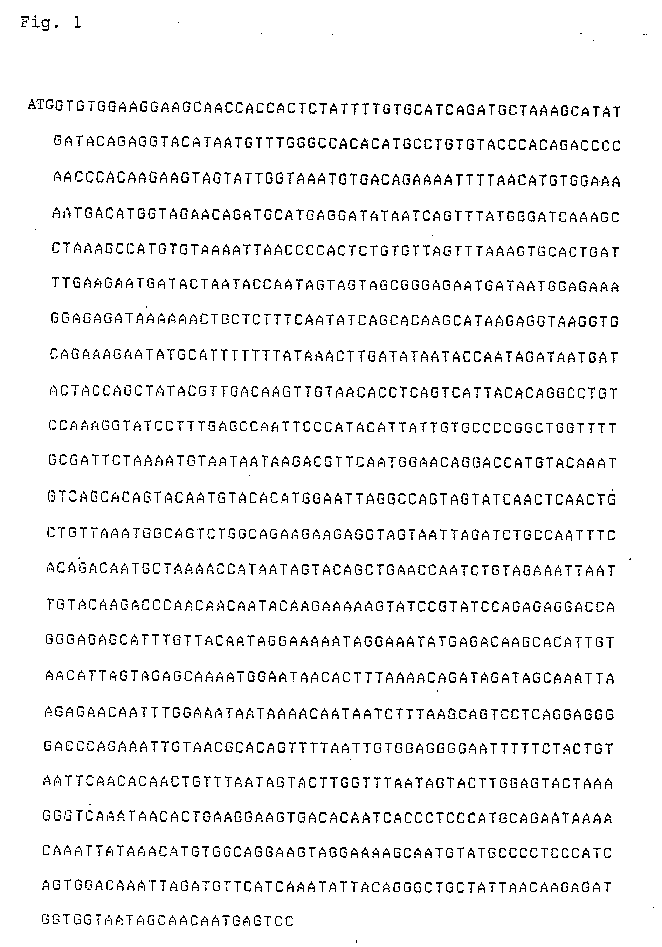

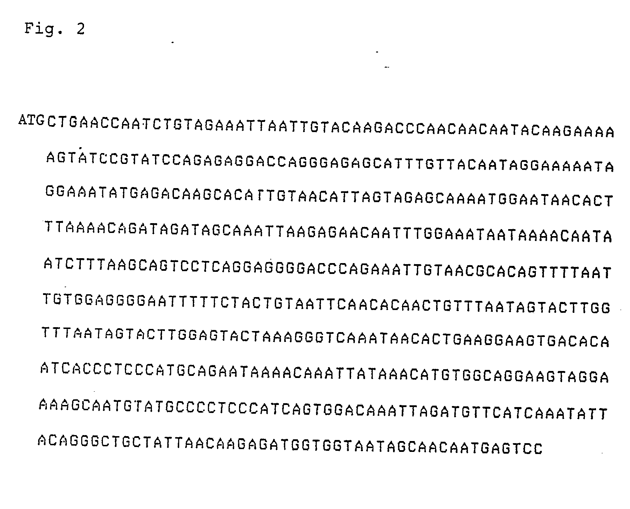

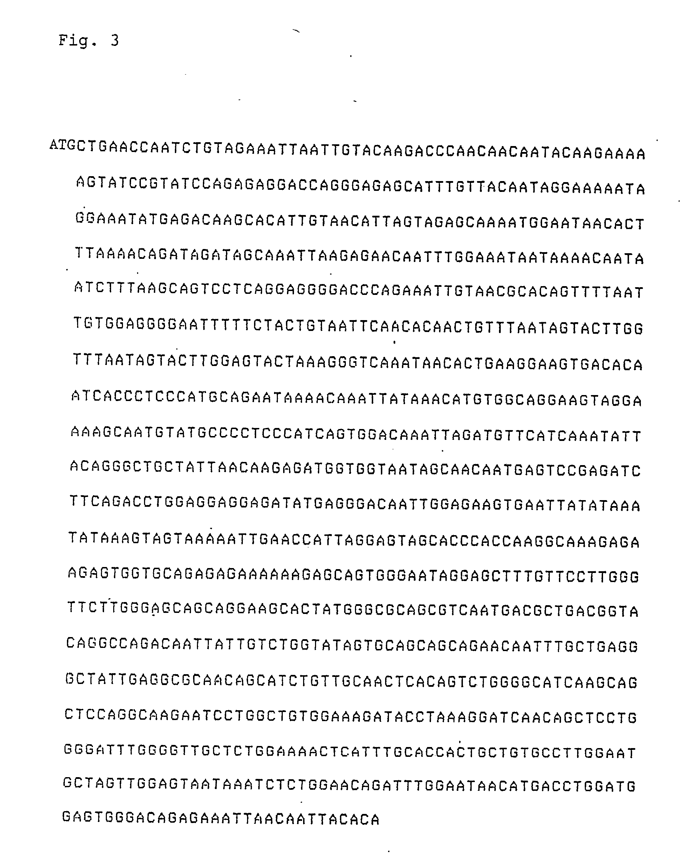

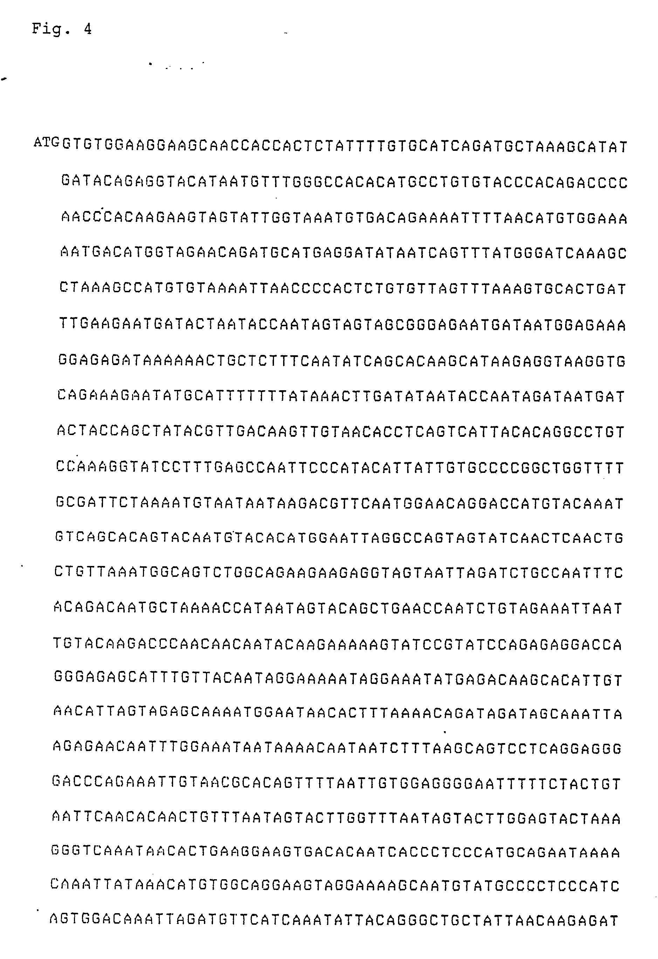

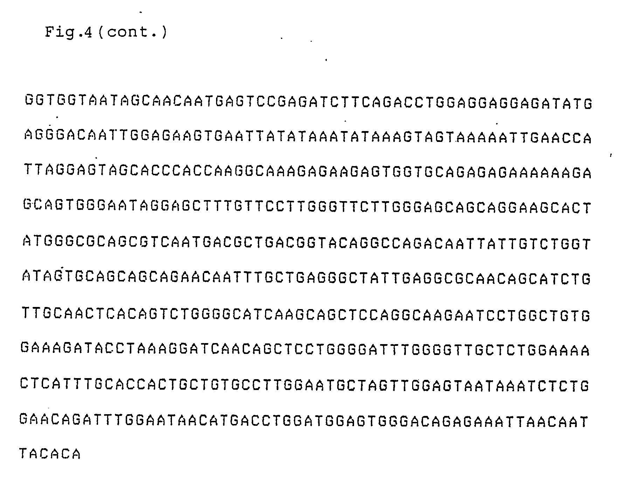

Novel proteins according to the present invention are the HIV portions of proteins R10, PB1, 590 and KH1, the amino-acid sequences of which are respectively given in claims 1, 2, 3 and 4, and also those sequences without the N-terminal methionine. A further aspect of the invention comprises DNA encoding the novel proteins, (examples of such nucleotide sequences are shown in the accompanying Figures 1, 2, 3 and 4 respectively) and transfer vectors comprising such DNA, e.g. plasmids pΔPB1 and pd2PB1.

The novel proteins can be used in assays for detecting or quantifying antibody against HIV, e.g. when attached to an immunoabsorbent solid phase such as is provided by a glass or plastic bead, a well of a microtitre plate or a test tube. The proteins may also be incorporated into vaccine compositions, as is also generally described in EP-A-0255190.

The following illustrates the present invention.

## Non-fusion Derivative of PB1

A non-fusion derivative of the PB1 protein, containing no non-HIV amino-acids other than an N-terminal methionine, was constructed using oligonucleotide-directed site-specific mutagenesis (Inouye, S. and Inouye, M., "Synthesis & Applications of DNA & RNA", ed. Narang, Saran A. Academic Press, 1987). In this procedure, 90 non-HTLV-III bp upstream and 39 downstream of the env gene sequence in pPB1 were deleted via DNA loopouts generated by hybridization with synthetic oligonucleotides.

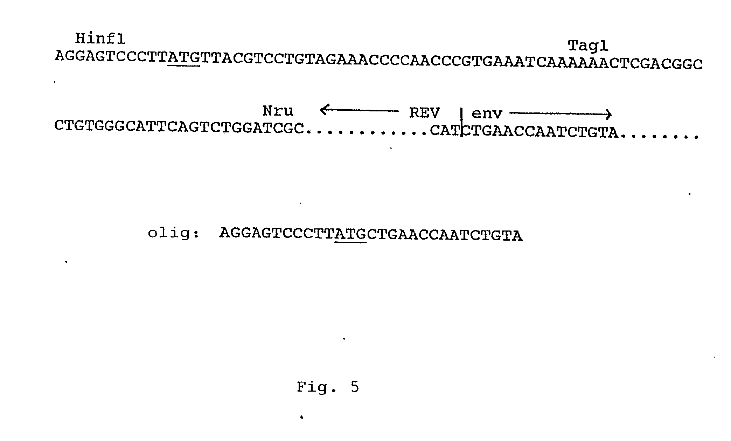

The oligonucleotide synthesized for the N-terminal loopout was designed so that the start codon of the β-glucuronidase gene is placed immediately adjacent to the 5′ end of the HTLV-III env gene sequence (Figure 5). The oligonucleotide includes sequences homologous to both sides of this newly created junction that allow proper hybridization to the plasmid DNA.

The two DNA molecules used to form a heteroduplex with a single-stranded gap that is the substrate for hybridization were created by digesting pPB1 with SalI and HpaI, or with PstI linearized pPB1, and a double digest with SalI and HpaI yields fragments of 3800 and 700 bp, the larger of which was gel-isolated for use in the mutagenesis.

Kinasing of the oligonucleotide, hybridization, polymerization and ligation to yield closed circular molecules were done according to the methods of Inuoye and Inouye mentioned above. To enrich for DNA molecules containing the deletion, the DNA mixture was digested with MluI, which cuts within the region being deleted.

The digested DNA was used to transform competent E. coli JM105 cells and plasmid-containing transformants were isolated by overnight growth on YT (8 g tryptone, 5 g yeast extract, and 5 g NaCl per liter) Cm plates at 37° C.

Plasmid DNA was isolated from each transformant and screened for the correct construction by simultaneous digestion with MluI and HindIII. Molecules that were not deleted yielded fragments of approximately 3900 and 600 bp. Those containing the deletion did not have the MluI site and yielded linear molecules of approximately 4400 bp. Plasmid DNA from transformants that appeared to contain the deletion was retransformed to ensure segregation of deleted and non-deleted plasmids and the recovery of pure plasmid populations. DNA from these second transformants was analyzed as in the previous digest and was determined to have the correct construction. This plasmid was designated pΔPB1.

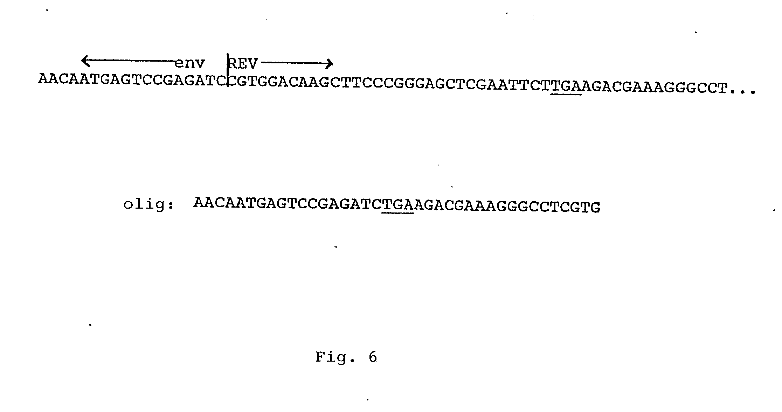

To eliminate the C-terminal non-HTLV-III amino acids, oligonucleotide-directed site-specific mutagenesis was carried out as above, using the pΔPB1 plasmid as a substrate. The oligonucleotide (Figure 6) was designed to position the TGA codon that occurs out-of-frame downstream from the env gene sequence so that it is immediately adjacent to the 3′ end of the env gene sequence and in-frame to act as a translational stop codon.

The molecules to form the heteroduplex used for hybridization were created by digesting pΔPB1 and PstI alone or with KpnI and HpaI. The large KpnI/HpaI fragment encompassing most of the vector was gel-isolated for use in the mutagenesis. Kinasing, hybridization, polymerization and ligation were performed as above. Enrichment for deleted molecules was accomplished by digesting with HindIII, which cuts within the region being deleted. The DNA was used to transform cells as above.

Plasmid DNA was isolated from each transformant and screened for the correct construction by simultaneous digestion with EcoRI and HpaI. The deleted plasmid yields two restriction fragments of 2900 and 1750 bp. Plasmid DNA showing this pattern was retransformed as above, and DNA from these transformants was analyzed with the same digest. This plasmid, containing N-terminal and C-terminal deletions, is designated pd2PB1.

When the strain harboring plasmid pΔPB1 is grown in 2% medium (2% yeast extract, bactotryptone, casamino acids [Difco, Detroit, MI], 0.2% potassium monobasic, 0.2% potassium dibasic, and 0.2% sodium dibasic) containing 50 μg/ml ampicillin and the total complement of cellular proteins electrophoresed on an SDS-polyacrylamide gel, a protein of approximately 22 kD can be visualized by either coomassie blue staining or by western blot analysis using as probe selected sera from animals immunized with recombinant env gene proteins. Under the same conditions, a protein of approximately 20 kD is produced in a strain containing pd2PB1.

The technique of oligonucleotide-directed site-specific mutagenesis can be used in a similar way to eliminate the non-HTLV-III amino acids flanking the env gene fusion proteins R10, 590, and KH1.

In the procedure detailed above, the removal of the non-HTLV-III sequence from the fusion proteins involves removal of amino acids at both the N-terminus and the C-terminus of the protein and is accomplished in two sequential steps.

It is well known in the art that a methionine at the N-terminal position can be enzymatically cleaved by the use of the enzyme methionine aminopeptidase (MAP). MAP has been cloned from E. coli (Ben-Bassat, A., Bauer, K., Chang, S.-Y., Myambo, K., Boosman, A. and Chang, S. [1987] Journal of Bacteriology 169(2): 751-757) and Salmonella typhimurium, and in vitro activity has been demonstrated on recombinant proteins (Miller, C.G., Strauch, K.L., Kukral, A.M., Miller, J.L., Wingfield, P.T., Massei, G.J., Werlen, R.C., Graber, P. and Movva, N.R. [1987] Proc. Natl. Acad. Sci. USA 84:2718-2722). Therefore, removal of an N-terminal methionine may be achieved either in vivo by expressing the protein in a host which produces MAP (e.g., E.coli CM89 or S. cerevisiae), or in vitro by use of purified MAP (e.g., procedure of Miller et al.).

## pd2PB1 Purification

Unless specified otherwise, all steps are carried out at room temperature.

Lysis--Three 700 ml bottles of frozen cell paste containing pd2PB1 are thawed at 37°C, and are then spun at 4,000 rpm in a J-6B centrifuge with a JS-4.2 rotor (Beckman, Palo Alto, CA) at 4°C for 30 min. The supernatant is then discarded and the weight of the cell pellet is determined. The cell pellet (typically 1 kg) is resuspended in 2 volumes of lysis buffer (v/w) which consists of 8 M urea, 20 mM Tris-HCl (pH 7.5 ± 0.1), 1 mM EDTA, 14.7 mM 2-mercaptoethanol and 1 mM PMSF.

The resuspended cell pellet is run through a Type TDK Pilot DYNO-MILL® (Impandex Inc., Maywood, NJ) containing 0.5-0.7 mm glass beads at 200-400 ml/min. Prior to use the DYNO-MILL® is charged with one liter of lysis buffer and cooled so that the solution flowing through is at less than ambient temperature. The resuspended cell pellet is passed through the DYNO-MILL® twice, and after the second pass, the DYNO-MILL® is washed with 1 liter of lysis buffer. Lysed cell suspension and wash are pooled.

### Concentration and filtration

The lysed cell suspension plus one liter wash is concentrated to 800 ml using a 0.45 μm. DURAPORE Pellicon cassette in a Pellicon 4 GPM system (Millipore, Bedford, MA). The concentration is done with an inlet pressure of less than or equal to 275 kPa and an outlet pressure between 70 and ]40 kPa. After concentration the lysed cell suspension is filtered with 4 liters of lysis buffer using the same Pellicon system, cassette and pressure settings with the tubing rigged for dyafiltration.

### Extraction

The washed lysis cell suspension is extracted with 10 l of extraction buffer consisting of 6 M guanidine

HCl, 100 mM Tris-HCl (pH 7.6 ± 0.1), and 10 mM EDTA, using the same Pellicon system, cassette and pressure settings as described above with the tubing rigged for dyafiltration.

Buffer exchange

The filtrate from the previous step is typically concentrated to 1 liter using a Pellicon 4GPM system with two PTGC cassettes (10,000 NMWL). The concentration is done with an inlet pressure of less than or equal to 345 kPa and an outlet pressure between 205 and 310 kPa. After concentration, the supernatant is buffer exchanged with CM column buffer consisting of 8 M urea, 25 mM potassium phosphate, and 1 mM EDTA (pH 6.8 ± 0.1), with conductivity less than or equal to 3.0 ms/cm. For buffer exchange, the same Pellicon system, the same cassettes and the same pressure settings as above are used with the tubing rigged for dyafiltration. Eight liters of CM column buffer are used to buffer exchange 1 liter of concentrated extract. After buffer exchange, the buffer-exchanged extract is drained from the system and the system is washed with 1 liter of CM column buffer. the buffer-exchanged extract and the wash are pooled and the solution's conductivity and pH are measured. The conductivity of the solution is adjusted to less than or equal to 3.0 ms/cm with deionized 8 M urea and the pH is adjusted to be within the range of 6.5-7.0.

CM chromatography

A 50 x 51 cm column of CM SEPHAROSE® FAST FLOW (Pharmacia, Piscataway, NJ) is equilibrated by washing the column sequentially with 4 column volumes of 0.5 M NaOH, 2 column volumes of deionized water and 2-3 column volumes of CM column buffer. The column is considered equilibrated when the pH of the outflow is within 0.2 units of the CM column buffer and the conductivity of the outflow is within 0.3 ms/cm of the CM column buffer.

For loading, the buffer exchanged extract is pumped on to the column at an inlet pressure between 10 and 15 psi. After loading, the CM column is washed with CM column buffer until the OD at 280 nm of the outflow is less than 0.1. The pd2PB1 is then eluted with an 8-liter linear gradient of 0-0.5 M NaCl in CM column buffer and collected in 100 ml fractions. The fractions are assayed by SDS-PAGE and Western with anti-gp160 antibody, and those containing significant pd2PB1 and trace contaminants are pooled.

Organic extraction

The pooled protein solution from the previous step is brought to a ratio of 55% acetonitrile to 45% protein solution (v/v) by the slow addition of pure acetonitrile with mixing. After addition of all of the acetonitrile, the solution is centrifuged in a J2-21 centrifuge using a JA10 rotor (Beckman) at 10,000 rpm and 4° C for 15 min. After centrifugation, the supernatant is collected and the pellet is discarded.

The centrifugation supernatant is brought to a ratio of 35% ethanol to 65% supernatant (v/v) by slow addition of 95% ethanol with mixing. After addition of all of the ethanol, the solution is centrifuged in a J2-21 centrifuge using a JA-10 rotor at 10,000 rpm and 4° C for 15 min. After centrifugation the pellet is collected and the supernatant is discarded.

The pellet is allowed to air dry for 15 min, and is then redissolved in S-300 column buffer, which consists of 8 M urea, 0.3 M glycine, 5 mM EDTA, 15 mM 2-mercaptoethanol, 1 mM dithiothreitol (DTT) (pH 8.50 ± 0.01). The pellet is dissolved in a volume of S-300 column buffer equal to one-tenth the volume of the pooled protein solution at the beginning of this step.

Concentration

The absorbance of the redissolved protein solution from above is determined at 280 nm and an approximate protein concentration is determined by assuming that a 1 mg/ml solution of protein has an absorbance of 1.0 at 280 nm. The solution is concentrated to 10 mg/ml using a 200 ml Amicon stirred cell concentrator with a YM-10 membrane.

S-300 chromatography

Thirty to seventy ml of the concentrated protein solution is loaded on a 5.0 x 135 cm column of SEPHACRYL® S-300 from Pharmacia. The column had been previously equilibrates with S-300 column buffer which consists of 8 M urea, 0.3 M glycine, 5 mM EDTA, 15 mM 2-mercaptoethanol, 1 mM DTT (pH 8.50 ± 0.01). After loading, the column is run isocratically in the same buffer. Twenty ml fractions are collected and the fractions are assayed for pd2PB1 content by SDS-PAGE.

Equal volume aliquots are taken from suitable fractions containing pd2PB1 and are used to determine which fractions are satisfactory for pooling. The aliquots are pooled, dialyzed overnight versus 8 M urea, 25 mM sodium phosphate, 1 mM EDTA (pH 6.8 ± 0.1), and the OD at 280 nm of the dialyzed pool is determined using the dialysis buffer as blank. The protein concentration of the solution is determined using the calculated extinction coefficient of pd2PB1 of 1.0 $(mg/ml)^{-1}$ SDS-PAGE is run on 10 µg of the dialyzed pooling using a 15% SDS acrylamide gel. After coomassie staining and destaining, the gel is scanned using an LKB (Gaithersburg, MD) scanning densitometer attached to a Waters (Milford, MA) 740 Integrator. If the pd2PB1 band on the gel is more than 97% pure, then the fractions that were used for the aliquot are checked for endotoxins at a 1 to 20 dilution in the Limulus Amebocyte Lysate (LAL) assay using 0.06 eu/ml tubes. If the LAL test on the diluted fractions is negative, the fractions are pooled and used for subsequent operations. If the gel fails to meet the purity specification, the process is repeated using equal volume aliquots from a different set of fractions. Only those fractions having a negative LAL test at a 1 to 20 dilution are pooled.

## Claims

1. A protein having the amino-acid sequence

MetValTrpLysGluAlaThrThrThrLeuPheCysAlaSerAspAlaLysAlaTyr
AspThrGluValHisAsnValTrpAlaThrHisAlaCysValProThrAspPro
AsnProGlnGluValValLeuValAsnValThrGluAsnPheAsnMetTrpLys
AsnAspMetValGluGlnMetHisGluAspIleIleSerLeuTrpAspGlnSer
LeuLysProCysValLysLeuThrProLeuCysValSerLeuLysCysThrAsp
LeuLysAsnAspThrAsnThrAsnSerSerSerGlyArgMetIleMetGluLys
GlyGluIleLysAsnCysSerPheAsnIleSerThrSerIleArgGlyLysVal
GlnLysGluTyrAlaPhePheTyrLysLeuAspIleIleProIleAspAsnAsp
ThrThrSerTyrThrLeuThrSerCysAsnThrSerValIleThrGlnAlaCys
ProLysValSerPheGluProIleProIleHisTyrCysAlaProAlaGlyPhe
AlaIleLeuLysCysAsnAsnLysThrPheAsnGlyThrGlyProCysThrAsn
ValSerThrValGlnCysThrHisGlyIleArgProValValSerThrGlnLeu
LeuLeuAsnGlySerLeuAlaGluGluGluValValIleArgSerAlaAsnPhe
ThrAspAsnAlaLysThrIleIleValGlnLeuAsnGlnSerValGluIleAsn
CysThrArgProAsnAsnAsnThrArgLysSerIleArgIleGlnArgGlyPro
GlyArgAlaPheValThrIleGlyLysIleGlyAsnMetArgGlnAlaHisCys
AsnIleSerArgAlaLysTrpAsnAsnThrLeuLysGlnIleAspSerLysLeu
ArgGluGlnPheGlyAsnAsnLysThrIleIlePheLysGlnSerSerGlyGly
AspProGluIleValThrHisSerPheAsnCysGlyGlyGluPhePheTyrCys
AsnSerThrGlnLeuPheAsnSerThrTrpPheAsnSerThrTrpSerThrLys
GlySerAsnAsnThrGluGlySerAspThrIleThrLeuProCysArgIleLys
GlnIleIleAsnMetTrpGlnGluValGlyLysAlaMetTyrAlaProProIle
SerGlyGlnIleArgCysSerSerAsnIleThrGlyLeuLeuLeuThrArgAsp
GlyGlyAsnSerAsnAsnGluSer

2. A protein having the amino-acid sequence

Met LeuAsnGlnSerValGluIleAsnCysThrArgProAsnAsnAsnThrArgLys SerIleArgIleGlnArgGlyProGlyArgAlaPheValThrIleGlyLysIle GlyAsnMetArgGlnAlaHisCysAsnIleSerArgAlaLysTrpAsnAsnThr LeuLysGlnIleAspSerLysLeuArgGluGlnPheGlyAsnAsnLysThrIle IlePheLysGlnSerSerGlyGlyAspProGluIleValThrHisSerPheAsn CysGlyGlyGluPhePheTyrCysAsnSerThrGlnLeuPheAsnSerThrTrp PheAsnSerThrTrpSerThrLysGlySerAsnAsnThrGluGlySerAspThr IleThrLeuProCysArgIleLysGlnIleIleAsnMetTrpGlnGluValGly LysAlaMetTyrAlaProProIleSerGlyGlnIleArgCysSerSerAsnIle ThrGlyLeuLeuLeuThrArgAspGlyGlyAsnSerAsnAsnGluSer

3. A protein having the amino-acid sequence

MetLeuAsnGlnSerValGluIleAsnCysThrArgProAsnAsnAsnThrArgLys
SerIleArgIleGlnArgGlyProGlyArgAlaPheValThrIleGlyLysIle
GlyAsnMetArgGlnAlaHisCysAsnIleSerArgAlaLysTrpAsnAsnThr
LeuLysGlnIleAspSerLysLeuArgGluGlnPheGlyAsnAsnLysThrIle
IlePheLysGlnSerSerGlyGlyAspProGluIleValThrHisSerPheAsn
CysGlyGlyGluPhePheTyrCysAsnSerThrGlnLeuPheAsnSerThrTrp
PheAsnSerThrTrpSerThrLysGlySerAsnAsnThrGluGlySerAspThr
IleThrLeuProCysArgIleLysGlnIleIleAsnMetTrpGlnGluValGly
LysAlaMetTyrAlaProProIleSerGlyGlnIleArgCysSerSerAsnIle
ThrGlyLeuLeuLeuThrArgAspGlyGlyAsnSerAsnAsnGluSerGluIle
PheArgProGlyGlyGlyAspMetArgAspAsnTrpArgSerGluLeuTyrLys
TyrLysValValLysIleGluProLeuGlyValAlaProThrLysAlaLysArg
ArgValValGlnArgGluLysArgAlaValGlyIleGlyAlaLeuPheLeuGly
PheLeuGlyAlaAlaGlySerThrMetGlyAlaAlaSerMetThrLeuThrVal
GlnAlaArgGlnLeuLeuSerGlyIleValGlnGlnGlnAsnAsnLeuLeuArg
AlaIleGluAlaGlnGlnHisLeuLeuGlnLeuThrValTrpGlyIleLysGln
LeuGlnAlaArgIleLeuAlaValGluArgTyrLeuLysAspGlnGlnLeuLeu
GlyIleTrpGlyCysSerGlyLysLeuIleCysThrThrAlaValProTrpAsn
AlaSerTrpSerAsnLysSerLeuGluGlnIleTrpAsnAsnMetThrTrpMet
GluTrpAspArgGluIleAsnAsnTyrThr .

4. A protein having the amino-acid sequence

Met ValTrpLysGluAlaThrThrThrLeuPheCysAlaSerAspAlaLysAlaTyr AspThrGluValHisAsnValTrpAlaThrHisAlaCysValProThrAspPro AsnProGlnGluValValLeuValAsnValThrGluAsnPheAsnMetTrpLys AsnAspMetValGluGlnMetHisGluAspIleIleSerLeuTrpAspGlnSer LeuLysProCysValLysLeuThrProLeuCysValSerLeuLysCysThrAsp LeuLysAsnAspThrAsnThrAsnSerSerSerGlyArgMetIleMetGluLys GlyGluIleLysAsnCysSerPheAsnIleSerThrSerIleArgGlyLysVal GlnLysGluTyrAlaPhePheTyrLysLeuAspIleIleProIleAspAsnAsp ThrThrSerTyrThrLeuThrSerCysAsnThrSerValIleThrGlnAlaCys ProLysValSerPheGluProIleProIleHisTyrCysAlaProAlaGlyPhe AlaIleLeuLysCysAsnAsnLysThrPheAsnGlyThrGlyProCysThrAsn ValSerThrValGlnCysThrHisGlyIleArgProValValSerThrGlnLeu LeuLeuAsnGlySerLeuAlaGluGluGluValValIleArgSerAlaAsnPhe ThrAspAsnAlaLysThrIleIleValGlnLeuAsnGlnSerValGluIleAsn CysThrArgProAsnAsnAsnThrArgLysSerIleArgIleGlnArgGlyPro GlyArgAlaPheValThrIleGlyLysIleGlyAsnMetArgGlnAlaHisCys AsnIleSerArgAlaLysTrpAsnAsnThrLeuLysGlnIleAspSerLysLeu ArgGluGlnPheGlyAsnAsnLysThrIleIlePheLysGlnSerSerGlyGly AspProGluIleValThrHisSerPheAsnCysGlyGlyGluPhePheTyrCys AsnSerThrGlnLeuPheAsnSerThrTrpPheAsnSerThrTrpSerThrLys GlySerAsnAsnThrGluGlySerAspThrIleThrLeuProCysArgIleLys GlnIleIleAsnMetTrpGlnGluValGlyLysAlaMetTyrAlaProProIle SerGlyGlnIleArgCysSerSerAsnIleThrGlyLeuLeuLeuThrArgAsp GlyGlyAsnSerAsnAsnGluSerGluIlePheArgProGlyGlyGlyAspMet ArgAspAsnTrpArgSerGluLeuTyrLysTyrLysValValLysIleGluPro

LeuGlyValAlaProThrLysAlaLysArgArgValValGlnArgGluLysArg

AlaValGlyIleGlyAlaLeuPheLeuGlyPheLeuGlyAlaAlaGlySerThr

MetGlyAlaAlaSerMetThrLeuThrValGlnAlaArgGlnLeuLeuSerGly

IleValGlnGlnGlnAsnAsnLeuLeuArgAlaIleGluAlaGlnGlnHisLeu

LeuGlnLeuThrValTrpGlyIleLysGlnLeuGlnAlaArgIleLeuAlaVal

GluArgTyrLeuLysAspGlnGlnLeuLeuGlyIleTrpGlyCysSerGlyLys

LeuIleCysThrThrAlaValProTrpAsnAlaSerTrpSerAsnLysSerLeu

GluGlnIleTrpAsnAsnMetThrTrpMetGluTrpAspArgGluIleAsnAsn

TyrThr

5. A protein according to any preceding claim, which does not have the N-terminal methionine.

6. DNA encoding a protein according to any preceding claim.

7. A recombinant DNA transfer vector comprising DNA according to claim 6.

8. A vector according to claim 7, which is plasmid pΔPB1 or plasmid pd2PB1.

9. An immunochemical assay for detecting or quantifying antibody against HIV in a liquid, which comprises employing a protein according to any of claims 1 to 5.

10. An immunoadsorbent, suitable for use in a solid phase immunochemical assay for antibody against HIV, comprising a solid phase to which is attached a protein according to any of claims 1 to 5.

11. An immunoadsorbent according to claim 10, further comprising a post-coat of animal protein,

12. A method of detecting antibody against HIV in a liquid, which comprises the steps of:

(a) incubating the liquid with an immunoadsorbent according to claim 10 or claim 11, under conditions which allow the antibody (if present) in the liquid to bind to the immunoadsorbent;

(b) separating the immunoadsorbent from the liquid;

and

(c) determining the presence of antibody bound to the immunoadsorbent.

13. A method according to claim 12, wherein step (c) comprises incubating the immunoadsorbent with a labelled antibody against antigen of the species from which the liquid is derived; separating the immunoadsorbent from the labelled antibody; and detecting the label associated with the immunoadsorbent.

14. A method according to claim 13, wherein the labelled antibody is a labelled protein according to any of claims 1 to 5, or labelled protein A.

15. A method according to claim 13, wherein the liquid is a human serum or plasma sample; the immunoadsorbent comprises a bead coated with the protein; and the labelled antibody is labelled anti-(human IgG) antibody.

16. A method according to claim 15, wherein the anti-(human IgG) antibody is an animal (e.g. goat) antibody and the serum or plasma sample is diluted with normal serum from an animal of the same species.

17. A kit for use in detecting antibody against HIV, as in any of claims 12 to 16, the kit comprising:

(a) an immunoadsorbent according to claim 10 or claim 11;

(b) labelled HIV antibody; and

(c) means for detecting the label.

18. A protein according to any of claims 1 to 5, for stimulating a lymphocyte proliferative response in humans.

19. A vaccine composition comprising one or more proteins having the antigenic properties of a protein according to any of claims 1 to 5.

Fig. 1

ATGGTGTGGAAGGAAGCAACCACCACTCTATTTTGTGCATCAGATGCTAAAGCATAT
GATACAGAGGTACATAATGTTTGGGCCACACATGCCTGTGTACCCACAGACCCC
AACCCACAAGAAGTAGTATTGGTAAATGTGACAGAAAATTTTAACATGTGGAAA
AATGACATGGTAGAACAGATGCATGAGGATATAATCAGTTTATGGGATCAAAGC
CTAAAGCCATGTGTAAAATTAACCCCACTCTGTGTTAGTTTAAAGTGCACTGAT
TTGAAGAATGATACTAATACCAATAGTAGTAGCGGGAGAATGATAATGGAGAAA
GGAGAGATAAAAAACTGCTCTTTCAATATCAGCACAAGCATAAGAGGTAAGGTG
CAGAAAGAATATGCATTTTTTTATAAACTTGATATAATACCAATAGATAATGAT
ACTACCAGCTATACGTTGACAAGTTGTAACACCTCAGTCATTACACAGGCCTGT
CCAAAGGTATCCTTTGAGCCAATTCCCATACATTATTGTGCCCCGGCTGGTTTT
GCGATTCTAAAATGTAATAATAAGACGTTCAATGGAACAGGACCATGTACAAAT
GTCAGCACAGTACAATGTACACATGGAATTAGGCCAGTAGTATCAACTCAACTG
CTGTTAAATGGCAGTCTGGCAGAAGAAGAGGTAGTAATTAGATCTGCCAATTTC
ACAGACAATGCTAAAACCATAATAGTACAGCTGAACCAATCTGTAGAAATTAAT
TGTACAAGACCCAACAACAATACAAGAAAAAGTATCCGTATCCAGAGAGGACCA
GGGAGAGCATTTGTTACAATAGGAAAAATAGGAAATATGAGACAAGCACATTGT
AACATTAGTAGAGCAAAATGGAATAACACTTTAAAACAGATAGATAGCAAATTA
AGAGAACAATTTGGAAATAATAAAACAATAATCTTTAAGCAGTCCTCAGGAGGG
GACCCAGAAATTGTAACGCACAGTTTTAATTGTGGAGGGGAATTTTTCTACTGT
AATTCAACACAACTGTTTAATAGTACTTGGTTTAATAGTACTTGGAGTACTAAA
GGGTCAAATAACACTGAAGGAAGTGACACAATCACCCTCCCATGCAGAATAAAA
CAAATTATAAACATGTGGCAGGAAGTAGGAAAAGCAATGTATGCCCCTCCCATC
AGTGGACAAATTAGATGTTCATCAAATATTACAGGGCTGCTATTAACAAGAGAT
GGTGGTAATAGCAACAATGAGTCC

Fig. 2

ATGCTGAACCAATCTGTAGAAATTAATTGTACAAGACCCAACAACAATACAAGAAAA
AGTATCCGTATCCAGAGAGGACCAGGGAGAGCATTTGTTACAATAGGAAAAATA
GGAAATATGAGACAAGCACATTGTAACATTAGTAGAGCAAAATGGAATAACACT
TTAAAACAGATAGATAGCAAATTAAGAGAACAATTTGGAAATAATAAAACAATA
ATCTTTAAGCAGTCCTCAGGAGGGGACCCAGAAATTGTAACGCACAGTTTTAAT
TGTGGAGGGGAATTTTTCTACTGTAATTCAACACAACTGTTTAATAGTACTTGG
TTTAATAGTACTTGGAGTACTAAAGGGTCAAATAACACTGAAGGAAGTGACACA
ATCACCCTCCCATGCAGAATAAAACAAATTATAAACATGTGGCAGGAAGTAGGA
AAAGCAATGTATGCCCCTCCCATCAGTGGACAAATTAGATGTTCATCAAATATT
ACAGGGCTGCTATTAACAAGAGATGGTGGTAATAGCAACAATGAGTCC

Fig. 3

ATGCTGAACCAATCTGTAGAAATTAATTGTACAAGACCCAACAACAATACAAGAAAA
AGTATCCGTATCCAGAGAGGACCAGGGAGAGCATTTGTTACAATAGGAAAAATA
GGAAATATGAGACAAGCACATTGTAACATTAGTAGAGCAAAATGGAATAACACT
TTAAAACAGATAGATAGCAAATTAAGAGAACAATTTGGAAATAATAAAACAATA
ATCTTTAAGCAGTCCTCAGGAGGGGACCCAGAAATTGTAACGCACAGTTTTAAT
TGTGGAGGGGAATTTTTCTACTGTAATTCAACACAACTGTTTAATAGTACTTGG
TTTAATAGTACTTGGAGTACTAAAGGGTCAAATAACACTGAAGGAAGTGACACA
ATCACCCTCCCATGCAGAATAAAACAAATTATAAACATGTGGCAGGAAGTAGGA
AAAGCAATGTATGCCCCTCCCATCAGTGGACAAATTAGATGTTCATCAAATATT
ACAGGGCTGCTATTAACAAGAGATGGTGGTAATAGCAACAATGAGTCCGAGATC
TTCAGACCTGGAGGAGGAGATATGAGGGACAATTGGAGAAGTGAATTATATAAA
TATAAAGTAGTAAAAATTGAACCATTAGGAGTAGCACCCACCAAGGCAAAGAGA
AGAGTGGTGCAGAGAGAAAAAAGAGCAGTGGGAATAGGAGCTTTGTTCCTTGGG
TTCTTGGGAGCAGCAGGAAGCACTATGGGCGCAGCGTCAATGACGCTGACGGTA
CAGGCCAGACAATTATTGTCTGGTATAGTGCAGCAGCAGAACAATTTGCTGAGG
GCTATTGAGGCGCAACAGCATCTGTTGCAACTCACAGTCTGGGGCATCAAGCAG
CTCCAGGCAAGAATCCTGGCTGTGGAAAGATACCTAAAGGATCAACAGCTCCTG
GGGATTTGGGGTTGCTCTGGAAAACTCATTTGCACCACTGCTGTGCCTTGGAAT
GCTAGTTGGAGTAATAAATCTCTGGAACAGATTTGGAATAACATGACCTGGATG
GAGTGGGACAGAGAAATTAACAATTACACA

Fig. 4

ATGGTGTGGAAGGAAGCAACCACCACTCTATTTTGTGCATCAGATGCTAAAGCATAT
GATACAGAGGTACATAATGTTTGGGCCACACATGCCTGTGTACCCACAGACCCC
AACCCACAAGAAGTAGTATTGGTAAATGTGACAGAAAATTTTAACATGTGGAAA
AATGACATGGTAGAACAGATGCATGAGGATATAATCAGTTTATGGGATCAAAGC
CTAAAGCCATGTGTAAAATTAACCCCACTCTGTGTTAGTTTAAAGTGCACTGAT
TTGAAGAATGATACTAATACCAATAGTAGTAGCGGGAGAATGATAATGGAGAAA
GGAGAGATAAAAAACTGCTCTTTCAATATCAGCACAAGCATAAGAGGTAAGGTG
CAGAAAGAATATGCATTTTTTTATAAACTTGATATAATACCAATAGATAATGAT
ACTACCAGCTATACGTTGACAAGTTGTAACACCTCAGTCATTACACAGGCCTGT
CCAAAGGTATCCTTTGAGCCAATTCCCATACATTATTGTGCCCCGGCTGGTTTT
GCGATTCTAAAATGTAATAATAAGACGTTCAATGGAACAGGACCATGTACAAAT
GTCAGCACAGTACAATGTACACATGGAATTAGGCCAGTAGTATCAACTCAACTG
CTGTTAAATGGCAGTCTGGCAGAAGAAGAGGTAGTAATTAGATCTGCCAATTTC
ACAGACAATGCTAAAACCATAATAGTACAGCTGAACCAATCTGTAGAAATTAAT
TGTACAAGACCCAACAACAATACAAGAAAAAGTATCCGTATCCAGAGAGGACCA
GGGAGAGCATTTGTTACAATAGGAAAAATAGGAAATATGAGACAAGCACATTGT
AACATTAGTAGAGCAAAATGGAATAACACTTTAAAACAGATAGATAGCAAATTA
AGAGAACAATTTGGAAATAATAAAACAATAATCTTTAAGCAGTCCTCAGGAGGG
GACCCAGAAATTGTAACGCACAGTTTTAATTGTGGAGGGGAATTTTTCTACTGT
AATTCAACACAACTGTTTAATAGTACTTGGTTTAATAGTACTTGGAGTACTAAA
GGGTCAAATAACACTGAAGGAAGTGACACAATCACCCTCCCATGCAGAATAAAA
CAAATTATAAACATGTGGCAGGAAGTAGGAAAAGCAATGTATGCCCCTCCCATC
AGTGGACAAATTAGATGTTCATCAAATATTACAGGGCTGCTATTAACAAGAGAT

Fig.4 (cont.)

GGTGGTAATAGCAACAATGAGTCCGAGATCTTCAGACCTGGAGGAGGAGATATG
AGGGACAATTGGAGAAGTGAATTATATAAATATAAAGTAGTAAAAATTGAACCA
TTAGGAGTAGCACCCACCAAGGCAAAGAGAAGAGTGGTGCAGAGAGAAAAAAGA
GCAGTGGGAATAGGAGCTTTGTTCCTTGGGTTCTTGGGAGCAGCAGGAAGCACT
ATGGGCGCAGCGTCAATGACGCTGACGGTACAGGCCAGACAATTATTGTCTGGT
ATAGTGCAGCAGCAGAACAATTTGCTGAGGGCTATTGAGGCGCAACAGCATCTG
TTGCAACTCACAGTCTGGGGCATCAAGCAGCTCCAGGCAAGAATCCTGGCTGTG
GAAAGATACCTAAAGGATCAACAGCTCCTGGGGATTTGGGGTTGCTCTGGAAAA
CTCATTTGCACCACTGCTGTGCCTTGGAATGCTAGTTGGAGTAATAAATCTCTG
GAACAGATTTGGAATAACATGACCTGGATGGAGTGGGACAGAGAAATTAACAAT
TACACA

```
Hinfl                                                            Tagl
AGGAGTCCCTTATGTTACGTCCTGTAGAAACCCCAACCCGTGAAATCAAAAAACTCGACGGC

                       Nru ←——— REV | env ———→
CTGTGGGCATTCAGTCTGGATCGC...........CAT|CTGAACCAATCTGTA........


        olig:  AGGAGTCCCTTATGCTGAACCAATCTGTA
```

Fig. 5

←――――env  REV――――→
AACAATGAGTCCGAGATCCGTGGACAAGCTTCCCGGGAGCTCGAATTCTTGAAGACGAAAGGGCCT...

olig:  AACAATGAGTCCGAGATCTGAAGACGAAAGGGCCTCGTG

Fig. 6